# EUROPÄISCHE PATENTSCHRIFT

(11) **EP 1 993 631 B1**
(45) Veröffentlichungstag und Bekanntmachung des Hinweises auf die Patenterteilung: **04.09.2013**
(21) Anmeldenummer: 07722047.3
(22) Anmeldetag: 14.03.2007
(51) Int. Cl.: A61M 1/34

(54) **REGENERIERBARES FILTER ZUR EXTRAKORPORALEN BEHANDLUNG PARTIKELHALTIGER FLÜSSIGKEITEN UND DEREN ANWENDUNG**
REGENERATABLE FILTER FOR EXTRACORPORAL TREATMENT OF LIQUIDS CONTAINING PARTICLES AND USE THEREOF
FILTRE POUVANT ÊTRE RÉGÉNÉRÉ UTILISÉ DANS LE TRAITEMENT EXTRACORPOREAL DE LIQUIDES CONTENANT DES PARTICULES ET LEUR UTILISATION

(30) Priorität: 14.03.2006 DE 102006012024
(43) Veröffentlichungstag der Anmeldung: 26.11.2008
(73) Patentinhaber: Adexter GmbH, 18059 Rostock (DE)
(72) Erfinder: HEINRICH, Hans-Werner, 17489 Greifswald (DE); KRUSCHKE, Peter, 17493 Greifswald (DE)
(74) Vertreter: Baumbach, Friedrich
(86) Internationale Anmeldenummer: PCT/DE2007/000476
(87) Internationale Veröffentlichungsnummer: WO 2007/104298

(56) Entgegenhaltungen:
- EP-A1- 0 139 949
- US-A- 5 679 231
- US-A1- 2004 220 508
- US-B1- 6 497 675

## Beschreibung

Die Erfindung betrifft ein regenerierbares Filter zur extrakorporalen Behandlung partikelhaltiger Flüssigkeiten und deren Anwendung. Anwendungsgebiet der Erfindung ist die Medizin, insbesondere die direkte Blutbehandlung, wie z.B. in US-A-5679231 beschrieben.

### Stand der Technik

Neben unterstützender Beeinflussung der Heilung von Krankheiten werden seit Jahrtausenden arzneimittelwirksame Stoffe verabreicht. Eine weitere Möglichkeit der therapeutischen Beeinflussung ist die Entfernung von schädlichen Substanzen aus dem Blut durch eine extrakorporale Blutbehandlung. Ausgangspunkt dieser Entwicklung ist der klassische Aderlass, der für mehr als zweitausend Jahren eine Standardtherapie für bestimmte Krankheiten darstellte. Neue Materialen und Technologien, sowie die Erkenntnisse der Blutgruppen-Forschung ermöglichten die Einführung der Hämodialyse in die klinische Anwendung vor mehr als 50 Jahren und führten zur Blutaustausch-Therapie, die später durch den Plasma-Austausch ersetzt wurde. Geringe Spezifität, hohe Kosten und Infektionsgefährdung beschränken die Anwendung des Plasma-Austausches.

Hämofiltration, Hämodiafiltration, Doppelfiltration und Plasma-Adsorption stellen Meilensteine in der Anwendung extrakorporaler Therapieverfahren (oder auch therapeutische Apherese) dar. Mit der Plasma-Adsorption konnten erstmals Stoffe aus dem Blut entfernt werden, die größer sind als Albumin. Für die Bindung hochmolekularer Stoffe im strömenden Blut oder Plasma werden unspezifische oder spezifische Faktoren genutzt.

Durch elektrostatische oder hydrophobe Wechselwirkungen zwischen der Matrix und den Blutbestandteilen werde heute routinemäßig LDL, Beta2-Mikroglobulin, Endotoxine, Immunglobuline und zirkulierende Immunkomplexe aus dem Blut entfernt.

Die spezifische Affinität von Protein A zum Fc-Rezeptor von IgG ermöglichte die Entwicklung von Immunadsorbern, die z.B. für die Abreicherung von IgG zur Behandlung schwerer Formen der rheumatoiden Arthritis (Prosorba®) eingesetzt werden.

Spezifische Erkennungssequenzen (Antikörper, Peptide) ermöglichen die Entfernung von Zielstrukturen mit eindeutig definierter Spezifität aus dem Blut. Sie werden u.a. verwendet für die Elimination von LDL (Therasorb®, LDL Lipopak®), Lp(a) (Lp(a) Lipopak®), Acetylcholin-Rezeptor-Antikörper (MedisorbaMG®), anti-ß1-adrenerger-Antikörper (Corafin®) oder Entzündungs-Mediatoren (EP 1163004).

Die Verwendung patienteneigener, dissoziierter lmmunkomplex-Bestandteile als Liganden für einen patientenspezifischen Immunadsorber (DE 19538641) ist eine Sonderform auf dem Weg immer gezielterer und personalisierter Therapie.

Bei allen kontinuierlichen Aphereseverfahren wird in einem extrakorporalen Kreislauf Blut aus einer peripheren Vene oder einem zentralvenösen Katheter mittels einer Blutpumpe, meistens mit einem Blutfluss von 60-120 ml/min kontinuierlich entnommen, und nach Entfernung des Pathogens über eine andere periphere Vene retransfundiert. Die Bereitstellung dieses intermittierend nutzbaren extrakorporalen Blutkreislaufs unterliegt ähnlichen Bedingungen wie bei der extrakorporalen Hämodialyse.

Bei den meisten Aphereseverfahren ist eine Primärtrennung von Plasma und Blutzellen vor der eigentlichen Plasmabehandlung erforderlich. Diese Primärtrennung kann sowohl mittels Zentrifugationsplasmaseparation als auch mittels Filtrationsplasmaseparation erfolgen. Bei beiden Verfahren sind sowohl Vor- als auch Nachteile zu berücksichtigen. Im wesentlichen ist die Filtrationsplasmaseparation in der Handhabung unkomplizierter und führt zu einem thrombozytenfreien Plasma. Der Nachteil ist die Bildung einer Sekundärmembran im Plasmafilter, welche die Filtrationseffektivität zeitlich begrenzt. Dagegen kann mittels der Zentrifugationsplasmaseparation eine nahezu unbegrenzte Menge Plasma ununterbrochen gewonnen werden. Nachteilig kann sich die geringe Thrombozytenkontamination des Plasmas auf die Sekundärtrennung auswirken. Der Filtratfluss beträgt bei der Primärtrennung in der Regel ca. 30% des Blutflusses (Plasmafluss ca. 20-30 ml/min). je nach Indikation wird meist das Ein- bis Zweifache des Plasmavolumens des Patienten behandelt. Bei Behandlung von einem bzw. von zwei Patientenplasmavolumina (Annahme eines Einkompartmentmodells ohne Rückvertellung, Synthese oder Katabolismus) kann pro Behandlung theoretisch eine maximale Reduktion des Pathogens auf 37 % bzw. 14 % des Ausgangswertes erreicht werden. Diese Werte werden allerdings in der Praxis meist nicht realisiert.

### Unselektive Plasmapherese (Plasmaaustausch)

Bei unselektivem Plasma-Austausch (plasma exchange) wird das Plasma im extrakorporalen Kreislauf mit Hilfe eines Membranplasmaseparators oder einer Zentrifuge von den Blutzellen getrennt, das gesamte Plasma wird verworfen und isovolämisch durch eine Elektrolytlösung plus Humanalbumin oder Frischplasma substituiert. Die Substitutionslösung wird mit den separierten Blutzellen vereinigt und dem Patienten re-infundiert. Der Vorteil des unselektiven Plasma-Austauschs liegt im einfachen Aufbau des extrakorporalen Kreislaufs, der generellen Anwendbarkeit des Verfahrens für alle der Apherese zugänglichen Pathogene, der Effektivität bei nicht genau bekannter Pathogenstruktur (z. B. bei Acetylcholinrezeptorantikörpernegativer Myasthenia gravis) und des relativ geringen extrakorporalen Volumens. Nachteile sind die Immunglobulin- und Gerinnungsfaktor-Depletion, die Gefahr einer Unverträglichkeit des substituierten Fremdeiweißes und einer hyperonkotischen Substitution sowie die potentielle Infektionsgefahr bei der Übertragung von Pathogenen mit der Substitutionslösung.

Aus den letztgenannten Gründen wird der unselektive Plasmaaustausch heute nur noch eingesetzt, wenn kein selektives Verfahren zur Verfügung steht (z. B. bei TIT, Chylomicronämie, antikörper-negativer Myasthenia gravis).

Membranplasmaseparatoren bestehen aus Hohlfasernmodulen mit synthetischen Membranen (z. B. Polyethylen oder Polysulfon). Die Oberfläche beträgt zwischen 0,2-0,5 m², die Porengröße 0,2-0,5 µm. Zur Überwachung des extrakorporalen Kreislaufs werden hierfür speziell entwickelte Geräte eingesetzt; alternativ ist auch die Verwendung von Geräten für die Hämoperfusion oder die Hämofiltration möglich.

### Selektive Plasmapherese

Bei der selektiven Plasmapherese wird aus dem über einen Plasmafilter separierten Plasma (Primärtrennung) in einem Sekundärkreislauf entweder durch einen weiteren Filtrationsprozess (Sekundärtrennung) oder durch Adsorption (immunologisch oder physikochemisch) oder durch Präzipitation das Pathogen entfernt und das gereinigte Plasma wieder dem Patienten zugeführt. Die selektive Plasmapherese erfordert spezielle Geräte, die sowohl den extrakorporalen Blutkreislauf als auch den Sekundärkreislauf überwachen.

### Doppelfiltration (Kaskadenfiltration, Membran-Differential-Filtration)

Dieses Verfahren verwendet nach Separation des Plasmas in einem Sekundärkreislauf einen zweiten Filter kleinerer Porengröße (Cut-off 25-40 nm). Ziel ist, Albumin möglichst quantitativ zurückzugewinnen, das höhermolekulare pathogene Protein dagegen im Sekundärfilter zurückzuhalten, der im sog. "deadend" Modus arbeitet (Verschluss des distalen Auslasses der Hohlfasern). Da dieses Verfahren nach Molekülgröße (Molekulargewicht und räumlicher Molekülkonformation) trennt, eignet es sich nur zur Entfernung von hochmolekularen Pathogenen wie IgM, LDL, Fibrinogen oder a-2-Makroglobulin. Indikationen sind daher z.B. Hyperviskositätssyndrom, M. Waldenström, Kryoglobulinämie und Hypercholesterinämie. Der Einsatz der Doppelfiltratationsplasmapherese zur Behandlung von Mikrozirkulationsstörungen wird als Rheopherese bezeichnet.

Vorteile dieses Verfahrens gegenüber dem unselektiven Plasma-Austausch bestehen darin, dass keine Substitutionslösung erforderlich ist und die selektive Entfernung besonders der rheologisch aktiven Eiweiße möglich ist, ohne dass es zu Störungen der Hämostase kommt. Nachteile sind die limitierte Kapazität des Sekundärfilters durch mögliche Verstopfung der Hohlfasern bei sehr hohen Ausgangswerten sowie mögliche, je nach Verfahren unterschiedliche Immunglobulinverluste.

### Immunadsorption

Unter Immunadsorption versteht man klinisch die Bindung immunologisch aktiver Moleküle an z.B. immobilisierte Aminosäuren, Peptide oder Proteine. Die auf Adsorption basierenden Verfahren entfernen entweder bestimmte Proteinklassen oder spezifisch einen pathogenen Antikörper. Verfahrenstechnisch wird umgekehrt auch die LDL-Bindung an Anti-Apoprotein B-Antikörper als LDL-Immunadsorption bezeichnet.

### Elimination von Lipoproteinen

Das Liposorber®-System (Fa. Kaneka, Osaka; Fa. Hospal, Planegg) basiert auf der Adsorption von LDL und Lp(a) aus dem Plasma an Dextransulfat/Zellulose (DSC). Der Mechanismus beruht auf einer elektrostatischen Wechselwirkung der negativ geladenen Sulfatgruppen des Dextransulfats mit dem positiv geladenen Apo B der beiden o. g. Lipoproteine. HDL, Immunglobuline und Albumin werden nur in geringem Maße adsorbiert.

Bei der HELP®-Apherese (Heparin induzierte extrakorporale LDL-Präzipitation, Einmalprodukt, Fa. Braun, Melsungen) werden LDL, Lp(a) und Fibrinogen bei saurem pH von 5,12 mittels Heparin im extrakorporalen Kreislauf aus dem Plasma gefällt und abfiltriert.

### Elimination von Immunglobulinen

Immunosorba®-System (Fa. Fresenius HemoCare, St.Wendel) verwendet als Ligand Staphylokokken-Protein-A mit Sepharose als Träger.

Prosorba®-System (Fa. Fresenius HemoCare, St.Wendel) verwendet als Ligand Staphylokokken-Protein-A mit einer Silica-Matrix als Träger

Beim Globaffin® (Fa. Fresenius HemoCare, St.Wendel) wird das synthetische Peptid-GAM® als Ligand an Sepharose CL-4B immobilisiert. Die Bindungseigenschaften entsprechen denen des Proteins A.

Coraffin® (Fa. Fresenius HemoCare, St.Wendel) entfernt spezifisch Autoantikörper gegen den ß1-adrenergen Rezeptor des Herzmuskels. Es handelt sich hiermit um ein indikationsspezifisches Verfahren.

Beim Ig-Therasorb®-Verfahren (Fa. Miltenyi Biotec, Teterow) werden polyklonale Anti-human-Immunglobulin Schafsantikörper auf Sepharose CL-4B immobilisiert.

Das System Immusorba® (Fa. ASAHI/Diamed, Köln) arbeitet mit nichtwiederverwendbaren Adsorbern auf der Basis von Tryptophan- (TR-350L) oder Phenylalanin-Liganden (PH-350L), welche an eine Polyvinylethanol-Gelmatrix gebunden sind.

### Kryofiltration

Bei der Kryofiltration (Fa. Asahi Medical, Tokyo; Fa. Diamed, Köln) wird in einem Membran-Differential-Filtrationsverfahren das separierte Plasma zur Präzipitation von Kryoglobulinen auf 4° C abgekühlt und nach Abtrennung der Präzipitate mit Hilfe eines Kryofilters nach Wiederaufwärmung auf Körpertemperatur reinfundiert.

### Vollblutapherese

Bei der Vollblutapherese werden mit Hilfe adsorbierender Substanzen (Aktivkohle, Austauscherharze), die sich in granulierter Form in einer Adsorberpatrone befinden, schädliche Substanzen im extrakorporalen Kreislauf direkt aus dem Blut mehr oder weniger selektiv entfernt. Sie gleicht der Aktivkohlehämoperfusion, die in der Intensivmedizin bei einer Reihe von Vergiftungen eingesetzt wird. Die Größe der Adsorberpatrone muss eine ausreichende Austauschfläche und Kontaktzeit des Adsorbens gewährleisten.

Die direkte Adsorption von LDL und Lp(a) aus Vollblut ermöglicht das DALI®-System (Direkte Adsorption von Lipoproteinen der Fa. Fresenius HemoCare, St.Wendel). Die einmal verwendbaren Adsorptionspatronen bestehen aus negativ geladenen Polyacrylatliganden, die auf Polyacrylamid immobilisiert sind und auf elektrostatischem Wege die atherogenen Lipoproteine binden.

Verfahren, Filtration und Adsorption können in unterschiedlicher Weise kombiniert werden. Matson, et al. (US 6,287,516) beschreibt ein Hämofiltrationssystem, das aus einem Blutfilter mit nachgeschaltetem Adsorber besteht. Das Ultrafiltrat aus dem Filter (Ausschluss MW # 50.000 Dalton) wird über ein Schlauchsystem in eine Adsorbereinheit gepumpt, wo die Sepsis-Mediatoren gebunden werden. Das so behandelte Ultrafiltrat kann durch ein weiteres Pumpen-Schlauch-Ventil-System mit dem primär gefilterten Blut vereint und dem Patienten reinfundiert werden:

Der Vorteil der spezifischen Adsorption liegt auf der Hand, werden doch nur die (pathogenen) Zielsubstanzen entfernt, ohne durch unspezifische Adsorption von "heilenden" Plasmabestandteilen die Normregulation negativ zu beeinflussen. Nachteil aller spezifischen Adsorptionsverfahren ist, dass die verwendeten Liganden, meist spezifische Antikörper, das Produkt verteuern. Das belastet die Gesamtbehandlungskosten insbesondere bei Krankheiten, die mehrere Tage oder Wochen therapiert werden müssen. In EP 0139949 wird ein Apparat für die Blutreinigung beschrieben, der in einem Gehäuse sowohl die plasmafiltrierenden Hohlfasern enthält, wie auch im Plasmaraum eine Adsorptionsmatrix. Ein sehr ähnliches Gerät beschreibt Shettigar (WO 93/02777). Beiden ist gemein, dass die adsorbierende Matrix nicht regeneriert werden kann. Die Möglichkeit der Regeneration wird ausdrücklich in US 6497675B1 vorgesehen. Nachteil ist hier, dass die in das Plasmafilter integrierte Adsorber-Einheit zwar regeneriert werden kann, dazu aber aus dem Gehäuse entfernt werden muss. Diese Manipulation verkompliziert das Verfahren und erhöht das Verwechslungsrisiko und damit die Gefahr der Übertragung von Krankheiten. Die regenerierte Adsorbereinheit wird in einen weiteren Arbeitsschritt in ein neues Plasmafiltergehäuse eingeschraubt.

### Erfindungsgemäße Lösung

Wünschenswert ist die Kombination der Vorteile von Membranen (emittieren keine Partikel, Porengröße frei wählbar, preisgünstig) mit denen der Partikel (große Variationsmöglichkeiten bezüglich Stoffklassen, Größe, Oberfläche, Aktivierung und Kopplung von Liganden) in einem Gehäuse, das sowohl die Regeneration der teuren fuktionalisierten Partikel erlaubt als auch gleichzeitig die Innenseiten der Plasmafiltermembranen vom Biofilm befreit, der üblicherweise die Verwendbarkeit der Membranen einschränkt.

Ziel der Erfindung ist eine Behandlungseinheit, vorzugsweise für vollblut, die sich durch einen einfachen Systemaufbau auszeichnet, so durch Wegfall von Pumpen und zusätzlichen Schlauchverbindungen, und die Vorteile von Membranen und Partikeln in sich vereinigt. Die erfindungsgemäße Vollblut-Behandlungseinheit soll höhere Blutflüsse (bis 160 ml/min) ermöglichen, die Behandlungszeit verkürzen und vor allem regenerierbar sein.

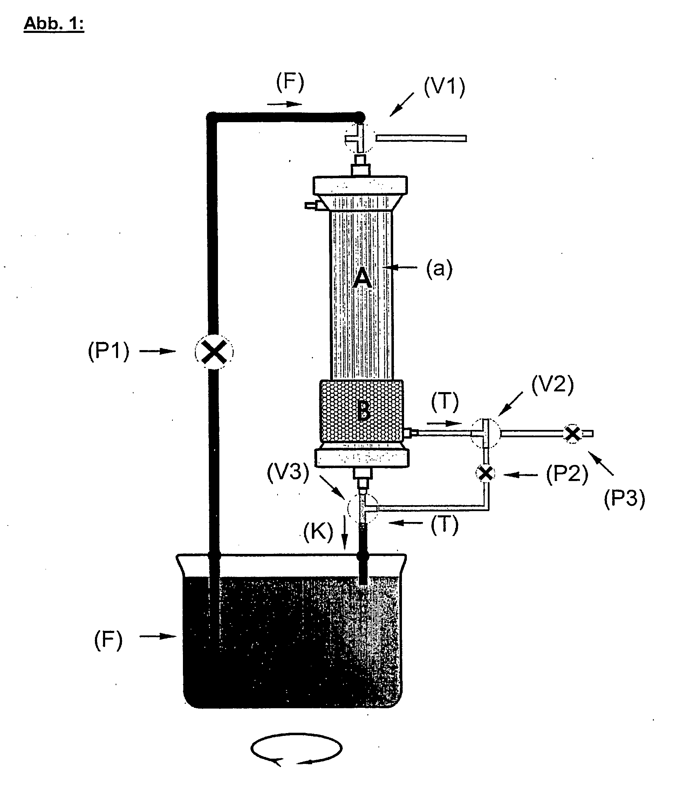

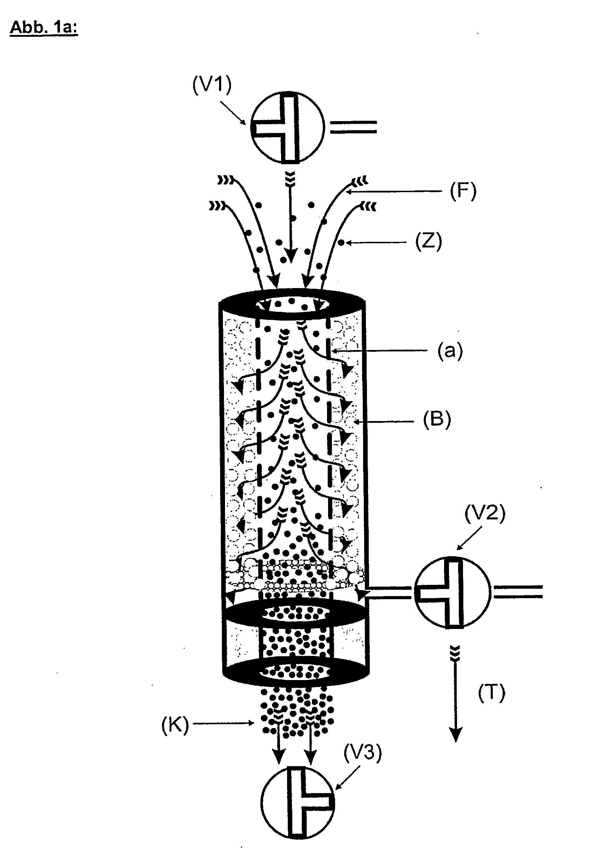

Die Zielstellung wird durch die Merkmale von Anspruch 1 erreicht. Vorzugsweise werden in einem in sich geschlossenen Gehäuse aus biokompatiblem Material übliche Plasmafilter (Membran-Hohlröhren) mit dem üblichen Porendurchmesser von 0,2 - 0,5 µm eingebracht (s. Abb 1 und Abb 1 a). Als Membranmaterial können Zellulose-Derivate oder synthetische Materialien wie z.B. Polysulfone oder Polyamide eingesetzt werden. Das Gehäuse dient gleichzeitig als Aufnahmebehältnis für die funktionalisierten Partikel. Als Material für die Partikel kann z.B. Polysulfon, Polyacrylonitril, Polmethylmethacrylat, PolyvinylAlkohol, Polyamide, Polycarbonate und Zellulose-Derivate verwendet werden. In das strömende Blut eingebracht, passiert das Plasma entsprechend dem Druckgefälle und der Porengröße die Membran. Außerhalb der Membran durchströmt nun das Plasma das Adsorber-Gel, bestehend aus unspezifisch oder spezifisch funktionalisierten Mikro-Partikeln mit einem Durchmesser oberhalb des Porendurchmessers der Membran. Das so spezifisch von bestimmten bioaktiven Stoffen gereinigte Plasma wird im Gehäuse mit dem intra-luminalen Plasmafilter-Blutstrom, vereinigt und als gereinigtes Vollblut dem Patienten reinfundiert. Ein Sieb verhindert dass Mikro-Partikel in den Blutstrom gelangen.

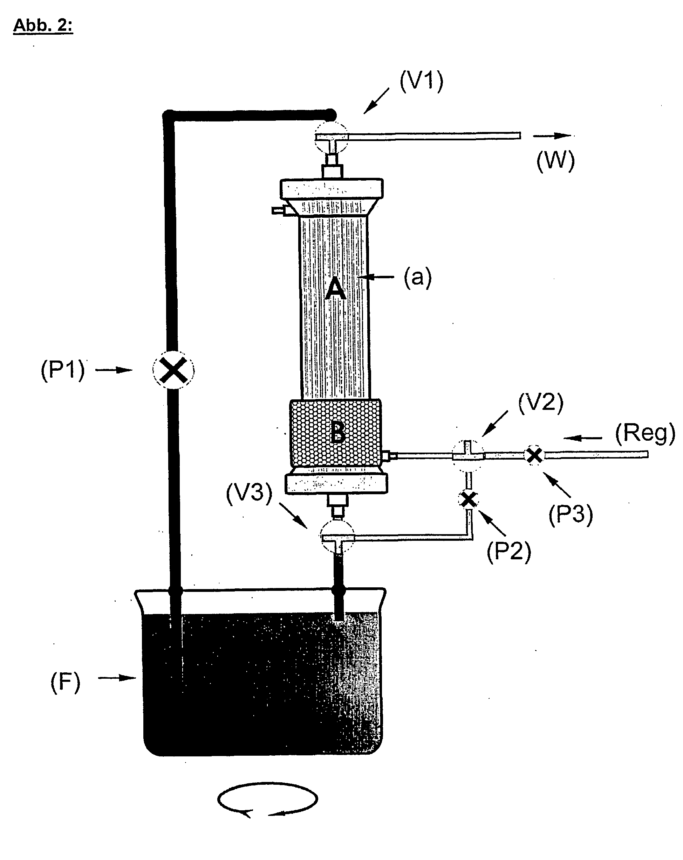

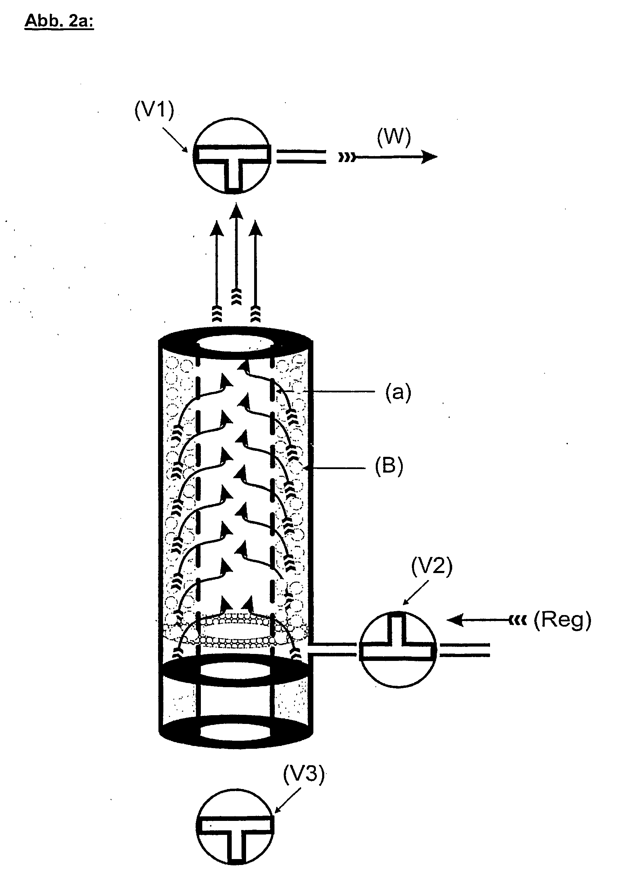

Erfindungswesentlich ist, dass im Filtersystem Dreiwegeventile so angeordnet sind, dass ein Einpumpen von Flüssigkeiten in den Partikelraum und ein Abfluss aus der Eintrittsöffnung ermöglicht wird (s. Abb. 2 und Abb. 2a). Durch Bedienung dieser Dreiwegeventile wird nach Erschöpfung des Adsorptionspotentials der Blut/Plasmastrom im Auslass des Gehäuses unterbunden. Die Regeneration erfolgt über das Partikelreservoir mit üblichen Puffern. Der Regenerationspuffer gelangt über die Poren des Plasmafilters in die Lumeninnenseite der Filtermembran und befreit die Oberfläche vom Biofilm, so dass nach Ablauf des jeweiligen Spülprogrammes das Filter für den nächsten Behandlungszyklus zur Verfügung steht. Erfindungsgemäß kann das Behandlungssystem auch aus zwei oder mehr Filtern bestehen, so dass während der Regeneration einer Adsorbereinheit die Behandlung mit einer anderen fortgesetzt wird und auf diesem Wege eine kontinuierliche Blutbehandlung stattfinden kann. S.S 9a

Die erfindungsgemäße Anordnung der Dreiwegeventile ermöglicht erstmalig eine Regeneration der funktionalisierten Partikel, ohne dass die Adsorber-Einheit aus dem Gehäuse entfernt werden muss. Auf diese Weise lässt sich der Regenerationsschritt schneller und mit geringerem technischen Aufwand durchführen. Das ist vor allem insofern von großer Bedeutung, als eine Verwechslung der Adsorbereinheiten verschiedener Patienten ausgeschlossen ist. Dieser Vorteil dürfte insbesondere beim Routinebetrieb im Krankenhaus von großem Wert sein. Außerdem besteht beim Einsatz der erfindungsgemäßen Vorrichtung keine Gefahr des Eindringens von Verunreinigungen in die Adsorbereinheit während des Auswechselns.

Zusammenfassend ergibt sich, dass durch den Einsatz von erfindungsgemäßen Vorrichtungendie Therapiekosten im Vergleich zu Einweg-Produkten die eine mehrere Tage lange extrakorporale Behandlung erfordern, deutlich gesenkt werden können.

Die Erfindung soll nachfolgend durch Ausführungsbeispiele näher erläutert werden.

Das erfindungsgemäße Filtersystem ist für Stoffabtrennungen aller Art aus flüssigen (extra-luminalen) Phasen geeignet, wo die partikelhaltige (intra-luminale) Phase in einem geschlossenem System nach Wiederzuführung der erfindungsgemäß behandelten flüssigen Phase weiter verwendet werden soll. Die adsorbierten Stoffe können im Anschluss mit geeigneten Flüssigkeiten vom Adsorbermaterial abgelöst und weiterverarbeitet bzw. verworfen werden. Darübe steht das Adsorbermaterial nach Regeneration für eine wiederholte Anwendung zur Verfügung.

Das Filtersystem ist ferner zur Behandlung von Blut in einem extrakorporalen Kreislauf einsetzbar, u.a.
- zur Abreicherung von Stoffen, die durch ihre Anwesenheit Krankheitszustände bewirken oder aufrechterhalten und
- zur Therapie von Krankheiten, die durch Störungen des angeborenen und/oder erworbenen Immunsystems ausgelöst oder aufrechterhalten werden.

### Ausführungsbeispiel 1

### Material und Methoden

- 3l Zitratblut boviner Herkunft wurden mit 1,5 µg rekombinantem humanen IL6 versetzt und über die in Abb. 1 dargestellte Vorrichtung im geschlossenen Kreislauf gepumpt (Parameter s.u.)
- Die experimentell genutzte Vorrichtung zur Entfernung von Antigenen aus Vollblut besteht aus einem Hollow-Fiber-Plasmaseparator (A), dessen unteres Gehäuseteil durch einen eingefügten Zylinder (B), in dem das Adsorbergel die Hohlfasern umgibt, ersetzt wurde. Das durch den Transmembrandruck freigesetzte Blutplasma muss den Adsorber passieren und wird dabei von den Zielsubstanzen befreit.
- Das Plasmaseparationsmodul bestand aus einem 0,4 m² Hohlfaserfilter (A).
- Der Adsorberbehälter enthielt 60 ml Sepharose, an die 5mg IgY (vitelline Antikörper aus den Eiern von Hühnern, die mit lL6 inokuliert wurden) pro ml Sepharose kovalent gebunden waren.
- Die Bindungskapazität des Adsorbers betrug 72 µg IL6.
- Die Blutflussrate wurde auf ca. 120 ml/min eingestellt.
- Ein Plasmastrom von 20-25 ml/min konnte dabei realisiert werden.
- Nach einem Plasmadurchsatz von insgesamt 1,5 l wurde die Adsorption beendet und die lL6-Konzentration im Blut bestimmt (human-IL6-ELISA, Milenia Biotec).

### Ergebnisse:

- Das Modul kann problemlos betrieben werden.
- Die behandelte Plasmamenge betrug 1,5 l.
- Hämolyse wurde nicht beobachtet.
- Die IL6-Anfangskonzentration von 500 pg/ml wurde während der 60 min Adsorptionsdauer auf 200 pg/ml gesenkt. Das entspricht einer Abreicherung von 60% bei einmaligem Durchlauf des Gesamtplasmavolumens durch den Adsorber entsprechend der in Abb. 1 dargestellten Vorrichtung (s. auch Abb 1 a).

### Ausführungsbeispiel 2

Aktivierung von Sepharose 4FF und Kopplung spezifischer anti-IL6-Antikörper Material und Methoden:
- Lösung A:: 10g Bromcyan (CNBr) werden in 100 ml Aceton gelöst
- Lösung B:: 15,2g Triethylamin (TEA) werden in 100 ml Aceton gelöst
- Lösung C:: 300 ml Aceton und 700 ml H₂O werden gemischt
- Lösung D:: 600 ml Aceton und 400 ml H₂O werden gemischt
- Puffer A:: 0,5 M NaCl in 0,2 M Carbonatpuffer, pH 8,5
- Puffer B:: 0,5 M NaCl in 0,1 M Acetat-Puffer pH 4,5
- Puffer C:: PBS pH 7,4

### Sepharose 4 FF (Amersham/GE)

50 mg vitelline Antikörper (IgY), spezifisch gegen humanes IL-6, gelöst in PBS, pH 8,5

### Durchführung:

- Aktivierung der Sepharose 4FF nach dem Cyanotransferverfahren *(*Kohn J and Wilchek M. The use of cyanogen bromide and other novel cyanylating agents for the activation of polysaccharide resins. Appl Biochem Biotechnol. 1984;9:285-305*.)* 15 ml der Sepharose in 20%igem Ethanol werden dem Originalbehälter entnommen und auf einer Fritte mit Wasser gewaschen (10x20 ml)
- Danach wird 5x mit 3:7 Aceton und danach mit 5x mit 6:4 Aceton gewaschen
- Die abgesaugte Sepharose wird in ein geeignetes Gefäß überführt und in 8 ml Aceton 6:4 suspendiert.
- Die Suspension wird bei -20°C gerührt (10 min)
- Dann werden 2 ml CNBr-Lösung zugesetzt, homogenisiert und 2 ml TEA-Lösung zugetropft
- Der Ansatz wird auf der Fritte abgesaugt und mit Eiswasser gewaschen
- Anschließend erfolgt die Aktivierungsbestimmung nach *(*Kohn J, Wilchek M. A colorimetric method for monitoring activation of Sepharose by cyanogen bromide. Biochem Biophys Res Commun. 1978;84(1):7-14*.),* indem die Konzentration der Cynatestergruppen auf der Sepharose ermittelt wird.
- Die trocken gesaugte aktivierte Sepharose wird in die IgY-Lösung gerührt und bei Raumtemperatur 2h geschüttelt
- Anschließend wird der Ansatz auf der Fritte abgesaugt und 2 mal mit Puffer A, 1:1 mit H₂O verdünnt, gewaschen. Die Lösungen werden gesammelt und der Proteingehalt durch Messung der UV-Adsorption bei 280 nm bestimmt.
- Das Gel wird dann einer mehrfachen Waschung mit den Puffern in der Reihenfolge A, C, B unterzogen. Die Beladung der Sepharose mit IgY wird danach in einem modifizierten micro BCA-Verfahren ermittelt. Die Kontrolle erfolgt über die UV-Proteinmessung der Waschflüssigkeiten.

### Ergebnisse:

- Aktivierung der Sepharose 9,8 µM/g feuchter Sepharose
- Proteinbeladung: 9,5 mg IgY/g feuchter Sepharose

### Ausführungsbeispiel 3

### Herstellung einer Vollblut-Behandlungseinheit

1. Herstellung eines Gehäuses zur Aufnahme handelsüblicher Plasmafiltrationsmembranen (0,3 m²) aus Hohlfasern und 20 ml mit anti-IL-6 funktionalisierten Adsorberpartikeln (Sepharose 4FF).
2. Einsetzen des Membranbündels und Vergießen beider Öffnungen zur luftdichten Verbindung mit dem Gehäuse. Die überstehenden Hohlfasermembranenden werden abgeschnitten.
3. Verschluss beider Enden mit Kappen, die Anschlussstutzen tragen.
4. Der obere Anschlussstutzen erhält ein Dreiwegeventil (V1), das dem Blutzufluss bzw. dem Regenerat- Abfluss dient.
5. Der untere seitliche Stutzen wird mit einem Dreiwegeventil (V2) versehen, das dem Abfluss des behandelten Plasmas bzw. dem Zufluss von Regenerierungs- und Waschlösungen dient.
6. Der untere Anschlussstutzen erhält das Dreiwegeventil (V3), das dem Zusammenführen des behandelten Plasmas mit den korpuskulären Blutbestandteilen zum behandelten Vollblut dient bzw. nach dessen Verschluss das Eindringen von Regenerierungsflüssigkeiten ins Vollblut verhindert.
7. Einfüllen der funktionalisierten Sepharose über den seitlichen Einfüllstutzen am Säulenkörper. Über den zweiten seitlichen Anschlussstutzen erfolgt die Entlüftung.

### Ausführungsbeispiel 4

### In-vitro-Abreicherung von IL6 aus Blut

Material und Methoden:
- Sepharose 4 FF (Amersham/GE), gekoppelt nach CNBr-Aktivierung mit vitellinen Antikörpern (IgY), die aus dem Eidotter mit humanem II-6 inokulierter Hühner gewonnen wurden
- Kopplungsdichte: 5mg IgY/ml gepackter Sepharose
- Recombinantes humanes IL-6 (R&D Systems GmbH)
- Heparinisiertes Vollblut
- IL6-ELISA-Kit (Milenia biotec)

### Durchführung:

- In drei verschiedenen Ansätzen wurden 2 ml Vollblut eines gesunden Menschen mit jeweils 146 ng recombinantem IL-6 gespiked
- Zu den Blutproben kamen jeweils 0,1 ml IgY-Sepharose, 5 mg/ml, spezifisch gegen humanes IL-6
- Die Proben wurden auf einem Taumler bei Zimmertemperatur bewegt
- Der IL-6-Gehalt des Blutes wurde zeitabhängig mittels ELISA bestimmt

### Ergebnisse:

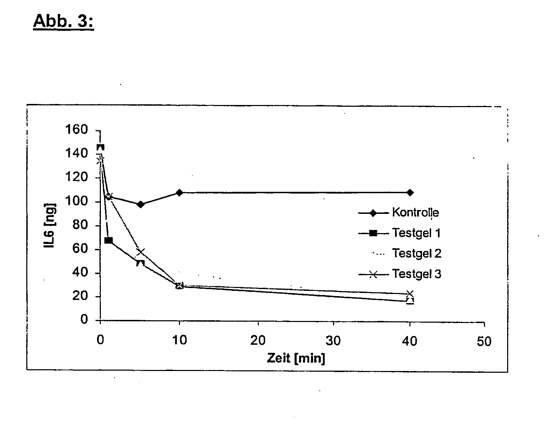

In Batch-Experimenten mit IL6 (Abb.3) konnte reproduzierbar nachgewiesen werden, dass Sepharose, beladen mit anti-IL-6-IgY (5mg/ml), in der Lage ist, IL6 aus dem Vollblut zu binden. Die eingesetzte IL-6-Menge entspricht etwa der, wie sie im Blut schwerkranker Patienten mit z.B. schwerer Sepsis und septischem Schock auftritt.

### Abbildungsbeschreibungen:

### Abb. 1: Adsorption

Die durch die Pumpe (P1) beförderte, partikelhaltige Flüssigkeit (F) tritt über ein Dreiwegeventil (V1), das sich in der Stellung 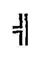 befindet, in die Vorrichtung AB ein. Infolge des bei der Passage der Hohlfasern (a) aufgebauten Transmembrandruckes tritt, bedingt durch die Porengröße der Membranen, ein partikelfreier Flüssigkeitsanteil in den außerhalb der Membranen befindlichen Raum aus. Diese Flüssigkeit (T) passiert den Teil B der Vorrichtung AB, der Partikel enthält, die eine spezifische Adsorption gelöster Stoffe ermöglichen (Details siehe Abb. 1 a). Über das Dreiwegeventil (V2),das sich in der Position 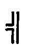 befindet und das Dreiwegeventil (V3), das in Position 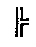 ist, werden die gereinigte Flüssigkeit (T) und das Partikelkonzentrat (K) vereint. Im Resultat des Prozesses wird die ursprüngliche Flüssigkeit (F) von gelösten, unerwünschten Bestandteilen befreit.
Die Behandlung kann diskontinuierlich und kontinuierlich verlaufen.

### Abb. 1a: Adsorption - Details

Die Poren der Membran (a) lassen aufgrund ihrer Größe nur den flüssigen Anteil (F) passieren. Dieser wird durch die Adsorberpartikel (B) von unerwünschten, löslichen Bestandteilen befreit und verlässt dann den Adsorber. Durch die dem Prozess entsprechende Schaltung der Dreiwegeventile (V2) und (V3) kann die Vereinigung mit dem am Durchfluss anfallenden Partikelkonzentrat (K) erreicht werden.

### Abb. 2: Regenerieren

Die Regeneration des Filters erfolgt, indem eine geeignete Waschlösung entgegengesetzt der Arbeitsrichtung des Filters gefördert wird.
Die von der Pumpe (P3) geförderte Waschlösung (Reg) gelangt über das Dreiwegeventil (V2) in den Adsorberraum B. Dort erfolgt die Reaktivierung der Adsorberpartikel. Danach durchspült die Flüssigkeit (Reg) die Hohlfasern (a) entgegen der Separationsrichtung und stellt dadurch die ursprüngliche Leistung wieder her (Details siehe Abb. 2a). Danach verlässt das mit Verunreinigungen beladene Medium (W) über das in Stellung 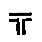 befindliche Dreiwegeventil (V1) den Adsorber, ohne Kontakt mit der zu reinigenden Flüssigkeit. Eine Kontamination der Flüssigkeit (F) im Reservoir wird durch die Stellung 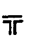 des Ventils (V3) verhindert.

### Abb. 2a: Regenerieren - Details

Das Adsorbermodul muss für die wiederholte Verwendung regeneriert werden. Dazu wird eine geeignete Flüssigkeit (Reg) über das Ventil (V2) entgegengesetzt der normalen Arbeitsrichtung in den Adsorber geleitet. In der 1. Phase erfolgt die desorptive Reaktivierung der Adsorberpartikel. Danach wird die Membran (a) gegensätzlich durchströmt und dabei von Ablagerungen in oder auf den Poren befreit. Filtrations- und Adsorptionsleistung des Adsorbermoduls werden wiederhergestellt. Die dem Prozess entsprechende Schaltung der Dreiwegeventile (V1-3) verhindert eine Kontamination des Flüssigkeitsvorrates mit (Reg) bzw. (W).

### Abb. 3: Adsorption von IL6 imBatch-Experiment

n=3, (Gelvolumen: 0,1 ml, Kopplungsdichte: 5 mg IgY/ml Gel, Medium: 2 ml heparinisiertes, humanes Vollblut, Antigenspike: 146 ng IL6, Test: IL6-ELISA-Kit

## Patentansprüche

1. Filtersystem zur membrangetrennten und adsorptiven Behandlung partikelhaltiger Flüssigkeiten, vorzugsweise Vollblut, bestehend aus
einem Gehäuse, in welchem
Hohlfasern so angeordnet sind, dass die Eintritts- und Austrittsöffnung für die Flüssigkeit außerhalb des Reaktionsgefäßes liegen und der Raum zwischen den Hohlfasermembranen und der Gehäuse-Innenwand zur Füllung mit adsorbierenden Mikropartikeln bestimmt ist, die größer sind als der Porendurchmesser der Hohlfasermembran, wobei sich vor der Austrittsöffnung
ein Sieb befindet, dass die adsorbierenden Mikropartikel in der Flüssigkeit von den flüssigen Bestandteilen trennt, vorzugsweise das die Mikropartikel von dem Plasmaraum trennt, **dadurch gekennzeichnet, dass**
Dreiwegeventile so angeordnet sind, dass ein Einpumpen von Flüssigkeiten in den Partikelraum und ein Abfluss aus der Eintrittsöffnung ermöglicht werden.

2. Filtersystem nach Anspruch 1, **dadurch gekennzeichnet, dass** die adsorptiv zu behandelnde partikelhaltige Flüssigkeit durch Filter mit einer Porengröße kleiner als die des Partikel-Durchmessers in eine partikelfreie, extra-luminale und partikelhaltige intra-luminale Phase getrennt werden.

3. Filtersystem nach den Ansprüchen 1-2, **dadurch gekennzeichnet, dass** die Hohlfaser-Membranen in ein Gehäuse eingebettet sind, das aus einem oder mehreren Kompartimenten bestehen kann.

4. Filtersystem nach den Ansprüchen 1-3, **dadurch gekennzeichnet, dass** die adsorbierenden Mikropartikel unspezifisch oder spezifisch funktionalisiert sind.

5. Filtersystem nach den Ansprüchen 1-4, **dadurch gekennzeichnet, dass** für die spezifische Funktionalisierung Proteine verwendet werden, die eine Zielsubstanz spezifisch binden.

6. Filtersystem nach den Ansprüchen 1-5, **dadurch gekennzeichnet, dass** die extra-luminale Phase mit den adsorbierenden Mikropartikeln in direkten Kontakt tritt und die Zielsubstanzen unspezifisch oder spezifisch an das Adsorbermaterial gebunden werden.

7. Filtersystem nach den Ansprüchen 1-6, **dadurch gekennzeichnet, dass** die von den Zielsubstanzen abgereicherte extra-luminale Phase über ein Dreiwegeventil mit der intra-luminalen Phase vereint wird.

8. Filtersystem nach den Ansprüchen 1-7, **dadurch gekennzeichnet, dass** über das Dreiwegeventil ein Einpumpen von Flüssigkeit in den extra-luminalen Adsorptionsraum und der Austritt aus der Filtereintrittsöffnung ermöglicht wird.

## Claims

1. Filter system for membrane-separated and adsorptive treatment of liquids containing particles, preferably full blood, consisting of a housing wherein hollow fibres are arranged in such a way that the inlet and outlet openings for the liquid are situated outside the reaction vessel and the volume between the hollow fibre membranes and the inner wall of the housing is intended for being filled with adsorbing microparticles which are bigger than the diameter of the pores of the hollow fibre membranes, wherein a screen is placed in front of the outlet opening separating the adsorbing microparticles in the liquid from the liquid components, preferably separating the microparticles from the plasma volume, **characterized by** an arrangement of the three-directional valves in such a way that a pumping of liquids into the particle space and a discharge from the inlet opening are made possible.

2. Filter system according to claim 1, **characterized in that** the liquid containing particles to be treated adsorptively by filters with a pore size smaller than the size of the diameter of the particles are separated into a particle-free, extra-luminal and particle-containing, intra-luminal phase.

3. Filter system according to claim 1 or 2, **characterized in that** the hollow fibre membranes are imbedded in a housing which can consist of one or several compartments.

4. Filter system according to any one of claims 1 - 3, **characterized in that** the adsorbing microparticles are functionalized non-specifically or specifically.

5. Filter system according to any one of claims 1 - 4, **characterized in that** proteins which specifically bind a target substance are used for the specific functionalization.

6. Filter system according to any one of claims 1 - 5, **characterized in that** the extra-luminal phase comes into direct contact with adsorbing microparticles and the target substances are bound non-specifically or specifically to the adsorbing material.

7. Filter system according to any one of claims 1 - 6, **characterized in that** the extra-luminal phase depleted of target substances is combined with the intra-luminal phase via the three-directional valve.

8. Filter system according to any one of claims 1 - 7, **characterized in that** the pumping of liquid into the extra-luminal adsorption volume and the discharge from the filter inlet opening are made possible via the three-directional valve.

## Revendications

1. Système de filtration pour le traitement de liquides à particules, de préférence du sang complet, avec séparation de membranes et par adsorption, composé d'un boîtier dans lequel des fibres creuses sont disposées de telle manière à ce que les orifices d'entrée et de sortie du liquide se trouvent en dehors du récipient de réaction et à ce que l'espace entre les membranes à fibres creuses et la paroi intérieure du boîtier soit destiné au remplissage de microparticules adsorbantes qui sont plus grandes que le diamètre des pores de la membrane à fibres creuses, un tamis devant l'orifice de sortie séparant les microparticules adsorbantes dans le liquide des composés liquides, séparant de préférence les microparticules de l'espace de plasma, **caractérisé par le fait que** les valves trois voies sont disposées de façon à permettre un pompage de liquides dans l'espace des particules et un écoulement depuis l'orifice d'entrée.

2. Système de filtration conformément à la revendication n°1, **caractérisé par le fait que** le liquide à particules à traiter par adsorption est séparé par un filtre ayant une grosseur de pore inférieure à celle du diamètre des particules dans une phase extra-luminale sans particules et une phase intra-luminale avec particules.

3. Système de filtration conformément aux revendications n°1 et n°2, **caractérisé par le fait que** les membranes à fibres creuses sont noyées dans un boîtier qui peut se composer d'un ou de plusieurs compartiments.

4. Système de filtration conformément aux revendications n°1 à n°3, **caractérisé par le fait que** les microparticules adsorbantes sont fonctionnalisées de façon non spécifique ou spécifique.

5. Système de filtration conformément aux revendications n°1 à n°4, **caractérisé par le fait que**, pour la fonctionnalisation spécifique, les protéines utilisées lient une substance cible de façon spécifique.

6. Système de filtration conformément aux revendications n°1 à n°5, **caractérisé par le fait que** la phase extra-luminale entre en contact direct avec des microparticules adsorbantes et que les substances cibles sont liées de façon non spécifique ou spécifique à la matière adsorbante.

7. Système de filtration conformément aux revendications n°1 à n°6, **caractérisé par le fait que** la phase extra-luminale enrichie des substances cibles est combinée à la phase intra-luminale par une valve trois voies.

8. Système de filtration conformément aux revendications n°1 à n°7, **caractérisé par le fait que** la valve trois voies permet un pompage de liquide dans l'espace d'adsorption extra-luminal et la sortie depuis l'orifice d'entrée du filtre.
